# EUROPÄISCHE PATENTSCHRIFT

(11) **EP 2 348 962 B1**
(45) Veröffentlichungstag und Bekanntmachung des Hinweises auf die Patenterteilung: **05.03.2014**
(21) Anmeldenummer: 09778684.2
(22) Anmeldetag: 23.09.2009
(51) Int. Cl.: A61B 5/00, B29C 45/14

(54) **VERFAHREN ZUR SPRITZGUSSHERSTELLUNG EINES MAGAZINS UND MAGAZIN FÜR EIN ANALYSEGERÄT**
METHOD FOR PRODUCING A MAGAZINE BY INJECTION MOLDING, AND A MAGAZINE FOR AN ANALYSIS DEVICE
PROCÉDÉ POUR PRODUIRE UN MAGASIN PAR MOULAGE PAR INJECTION, ET CHARGEUR POUR UN DISPOSITIF D'ANALYSE

(30) Priorität: 13.10.2008 EP 08166497
(43) Veröffentlichungstag der Anmeldung: 03.08.2011
(73) Patentinhaber: Roche Diagnostics GmbH, 68305 Mannheim (DE); F. Hoffmann-La Roche AG, 4070 Basel (CH)
(72) Erfinder: FREY, Stephan-Michael, 64347 Griesheim (DE); LIST, Hans, 64754 Hesseneck-Kailbach (DE); RITTINGHAUS, Andrea, 69239 Neckarsteinach (DE); SCHWEDE, Maik, 07747 Jena (DE)
(74) Vertreter: Durm & Partner
(86) Internationale Anmeldenummer: PCT/EP2009/006885
(87) Internationale Veröffentlichungsnummer: WO 2010/043300

(56) Entgegenhaltungen:
- WO-A-2007/041244
- US-A1- 2006 064 035

## Beschreibung

Die vorliegende Erfindung betrifft ein Verfahren zur Spritzgussherstellung eines Magazins für ein Analysegerät. Das Magazin hat eine Mehrzahl von Kammern, die benachbart zueinander angeordnet und durch Trennwände voneinander getrennt sind. Sie weisen eine Kammeraußenwand auf, welche von einer Außenwand des Magazins gebildet wird. Die Kammern sind insbesondere geeignet, um Analyseelemente aufzunehmen, mittels denen ein Analyt in einer Körperflüssigkeitsprobe bestimmt werden kann.

Auf vielen Gebieten der medizinischen Analyse werden Analyten in einer Körperflüssigkeitsprobe, insbesondere im Blut, bestimmt, die durch einen Stich in die Haut des Patienten gewonnen wird. Bei der Entnahme der Körperflüssigkeit aus einem Körperteil, vorzugsweise aus der Fingerbeere, reichen geringe Mengen aus, um einen Analyten, beispielsweise den Glucosegehalt im Blut, für medizinische und diagnostische Zwecke zu bestimmen. Die dabei verwendeten Geräte können auch von Laien problemlos verwendet werden.

Derartige Geräte werden beispielsweise von Diabetikern verwendet, um regelmäßig ihren Blutzuckergehalt zu überprüfen. Damit die Überwachung von dem Patienten auch tatsächlich mehrmals täglich durchgeführt wird, ist neben einer Schmerzarmut des Einstichs ein einfaches und komfortables Handling der Geräte notwendig. Neuere Geräteentwicklungen umfassen deshalb integrierte Analysesysteme, die ein "one-step-treatment" ermöglichen. Der Benutzer muss ein derart automatisiertes Gerät nur einmal ansetzen und kann dann - ohne weitere Handlungsschritte - das Analyseergebnis ablesen.

Analyseelemente, z.B. von integrierten Geräten, rufen eine messbare Änderung hervor, sobald sie mit einer den gesuchten Analyten enthaltenen Flüssigkeit in Berührung kommen. Diese Änderung kann elektrochemisch gemessen werden, wobei zwei Elektroden mit einer Probenaufnahmefläche des Analyseelements in Kontakt stehen. Die Messung einer Stromänderung lässt Rückschlüsse auf das Vorhandensein und die Menge eines gesuchten Analyten zu.

Neben der elektrochemischen Messung haben sich optische Messungen durchgesetzt. Die Kontaktierung der Probenflüssigkeit mit dem Analyseelement führt zu einer optisch messbaren Änderung, die ein Maß für das Vorhandensein und die quantitative Menge eines vorhandenen Analyten ist. Eine optische Auswerteeinheit muss dabei so positioniert sein, dass sie das Analyseelement abtasten kann, um die messbare Änderung zu detektieren. Dabei müssen gegebenenfalls das Analyseelement und die optische Messeinrichtung relativ zueinander bewegt werden.

Da die Analyseelemente vor der Analyse trocken gelagert sein müssen, sind sie in der Regel in Magazinen angeordnet, häufig auch in kombinierten Magazinen, die auch die Stechelemente einschließen. Derartige Magazine sind z.B. aus der WO 2007/041244 A2 oder der US 2006/0064035 A1 bekannt. Um eine optische Messung durchzuführen, kann das Analyseelement nach der Probenaufgabe in eine Position außerhalb des Magazins bewegt werden, so dass eine ungehinderte optische Abtastung möglich ist. Eine Bewegung des Analyseelements, zusätlich zu dem Stechelement ist mechanisch jedoch aufwendiger. Deshalb wird das Analyseelement bevorzugt in dem Magazin belassen und die Messoptik so positioniert, dass eine optische Erfassung durch die Magazinwand hindurch möglich ist. Das Magazin muss dabei möglichst transparent ausgebildet sein, damit keine Störungen der optischen Auswertung stattfinden, die das Messergebnis beeinflussen könnten.

Aufgabe der vorliegenden Erfindung ist es deshalb, ein Magazin mit verbesserten Eigenschaft, insbesondere ein Magazin mit optimierten optischen Eigenschaften, vorzuschlagen, das eine optische Auswertung eines Analyseelements in dem Magazin ermöglicht.

Gelöst wird die vorliegende Aufgabe durch ein Verfahren zur Spritzgussherstellung eines Magazins mit den Merkmalen des Anspruchs 1 und durch ein Magazin mit den Merkmalen des Anspruchs 10.

Mit dem erfindungsgemäßen Verfahren zur Spritzgussherstellung wird ein Magazin mit einer Mehrzahl von Kammern, die bevorzugt gleichgerichtet orientiert sind, angegeben. Die Kammern des Magazins sind durch Trennwände voneinander getrennt. Sie weisen eine Kammeraußenwand auf, welche von der Außenwand des Magazins gebildet wird. Zur Herstellung eines derartigen Magazins wird eine Gussform verwendet, die eine Angussstelle mit einer Angussöffnung, einen Außenwandkanal und Trennwandkanäle hat. Der Außenwandkanal der Gussform entspricht der Außenwand des herzustellenden Magazins. Die Trennwandkanäle entsprechen den Trennwänden zwischen den Kammern des Magazins. Die Trennwandkanäle sind derart orientiert, dass sie sich von der Angussstelle zu dem Außenwandkanal erstrecken.

Das erfindungsgemäße Verfahren umfasst die folgenden Schritte:
Eine Kunststoffmasse, die wenigstens nach der Aushärtung transparent ist, wird in die Angussöffnung der Gussform eingespritzt. Dies geschieht mittels Spritzgusstechnik mit einem vorbestimmten Druck und einer definierten Temperatur. Der Druck und die Temperatur sind in der Regel von der verwendeten Kunststoffmasse abhängig. In einem weiteren Schritt wird die Kunststoffmasse als Kunststoffmassenstrom von der Angussstelle durch die Trennwandkanäle in den Außenwandkanal derart strömen gelassen, dass die Kunststoffmassenströme in alternierenden Trennwandkanälen eine unterschiedliche Strömungsgeschwindigkeit haben. Zumindest ist die Strömungsgeschwindigkeit in zwei benachbarten Kanälen unterschiedlich, wobei Geschwindigkeit in jedem zweiten Kanal bevorzugt wiederum gleich ist. Die Kunststoffmasse erreicht somit das Ende der Trennwandkanäle unterschiedlich schnell, abhängig von der dort vorherrschenden Strömungsgeschwindigkeit.

In einem weiteren Schritt fließen die Kunststoffmassenströme am Ende der Trennwandkanäle in dem Außenwandkanal derart zusammen, dass sich an der Außenseite des Außenwandkanals eine Bindenaht ausbildet. Diese Bindenaht, die auch Fließnaht genannt wird, entsteht durch das Zusammentreffen zweier Kunststoffmassenströme. Die Bindenaht ist nicht nur an der Außenseite des Außenwandkanals ausgebildet, sondern bildet eine Ebene, aus der die Kunststoffströme zusammentreffen. Die Flächennormale der Ebene ist entweder (im Wesentlichen) senkrecht zu den Trennwandkanälen oder - bei trommelförmigen Gussformen - senkrecht zu einer sich radial erstreckenden Linie ausgerichtet.

Durch die Steuerung der Strömungsgeschwindigkeiten in den Trennwandkanälen lässt sich der Ort des Zusammentreffens zweier Kunststoffmassenströme beliebig festlegen. Wichtig ist dabei, dass die Bindenaht nicht in dem Bereich des Außenwandkanals ausgebildet wird, der für eine optische Messung einer Messgröße im Inneren des Magazins verwendet wird. Deshalb wird die Strömungsgeschwindigkeit der Kunststoffmassenströme derart eingestellt, dass sie in den benachbarten Kanälen unterschiedlich ist und die Bindenaht nicht im mittigen Bereich der Kammeraußenwand des Magazins ausgebildet wird. Die Bindenaht ist folglich jedenfalls nicht in der Mitte zwischen zwei benachbarten Trennwandkanälen angeordnet. Sie können aber in den Randbereichen zwischen zwei benachbarten Trennwandkanälen angeordnet werden, so dass die Bindenähte am Rand (Randbereich) der Kammeraußenwand des Magazins positioniert sind. Auch können die Bindenähte am Übergang zum Trennwandkanal liegen. Wichtig ist, dass ein transparenter Bereich in der Kammeraußenwand des Magazins zwischen zwei benachbarten Trennwandkanälen nicht durch eine Bindenaht gestört wird. Bevorzugt ist deshalb ein transparenter Bereich, der mittig zwischen zwei benachbarten Trennwandkanälen angeordnet ist, frei von Bindenähten.

Bevorzugt fließen die Kunststoffmassenströme in dem Außenwandkanal derart zusammen, dass die Bindenaht mit einem Trennwandkanal fluchtet. Die Bindenähte sind folglich so positioniert, dass sie im Bereich der Breite des Trennwandkanals, beispielsweise mittig oder außermittig zum Trennwandkanal, angeordnet sind. Sie können jedoch auch an der Grenze bzw. an dem Übergang zwischen Trennwandkanal und dem Bereich des Außenwandkanals, der später der Kammeraußenwand des Magazins entspricht, angeordnet sein.

In einer bevorzugten Ausführungsform sind die Strömungsgeschwindigkeiten in zwei benachbarten Trennwandkanälen derart eingestellt, dass sich die Bindenaht am Ende des Trennwandkanals ausbildet, in dem die Strömungsgeschwindigkeit geringer ist.

Ein wesentliches Merkmal zur Bestimmung der Position der Bindenaht an dem Außenwandkanal ist folglich die unterschiedliche Strömungsgeschwindigkeit des Kunststoffmassenstroms in den Kanälen. Sie lässt sich auf unterschiedliche Arten beeinflussen. Beispielsweise kann die Kunststoffmasse in einzelnen Trennwandkanälen mit einem höheren Druck eingespritzt werden als in anderen Trennwandkanälen. Die Strömungsgeschwindigkeit lässt sich auch durch die Eigenschaften der Kunststoffmasse variieren. In der Regel hängt die Fließ- bzw. Strömungsgeschwindigkeit eines Kunststoffes stark von seiner Temperatur ab. So könnten z.B. die durch einen bestimmten Trennwandkanal fließenden Kunststoffmassen wärmer sein als die durch einen anderen Kanal fließenden Massen. Hierdurch würde es zu einer Erhöhung der Strömungsgeschwindigkeit kommen. Die Erwärmung einzelner Kunststoffmassenströme in bestimmten Trennwandkanälen könnte beispielsweise durch ein zusätzliches Heizen einzelner Trennwandkanäle erzielt werden. Beispielsweise könnte ein Heizdraht in der Nähe der Kanäle angebracht werden. Ein Heizen durch Infrarotstrahlung, eine andere Strahlung oder andere Heizquellen ist ebenfalls denkbar.

Ein weiterer Parameter, um die Strömungsgeschwindigkeit in den Trennwandkanälen zu beeinflussen und zu steuern, ist die Geometrie der Trennwandkanäle. Bevorzugt sind in der Gussform deshalb benachbarte Trennwandkanäle alternierend weiter und enger. Folglich wechseln sich ein weiterer und ein engerer Kanal ab. Die Strömungsgeschwindigkeit in dem weiteren Trennwandkanal, in dem der Strömungswiderstand geringer ist, ist somit höher.

Die Begriffe "weiter" und "enger" beziehen sich nur auf eine Raumrichtung. In einer anderen Raumrichtung können die Kanäle durchaus andere Dimensionen aufweisen, beispielsweise kann der in der entscheidenden Raumrichtung (quer zur Strömungsrichtung zwischen den Seitenwänden der Trennwandkanäle) weitere Trennwandkanal in einer anderen Raumrichtung durchaus enger sein als sein benachbarter Kanal bzw. umgekehrt. Wichtig ist nur, dass durch den weiteren Trennwandkanal die Kunststoffmasse schneller strömen kann als durch den engeren Trennwandkanal.

Sind die benachbarten Trennwandkanäle weiter bzw. enger ausgebildet, so bildet sich die Bindenaht der Kunststoffströme im Außenwandkanal im Bereich des engeren Trennwandkanals aus. Die Bindenähte fluchten folglich mit dem engeren Trennwandkanal. Dabei befinden sich die Bindenähte an der Außenseite des Außenwandkanals innerhalb eines Bereiches, der sich in der Verlängerung der Trennwand des engeren Trennwandkanals ergibt.

Darüber hinaus ist es auch denkbar, die Trennwandkanäle an den Seiten zu beschichten, um den Fließwiderstand zu erhöhen oder zu verringern, um so Einfluss auf die Fließgeschwindigkeit zu nehmen.

In einer bevorzugten Ausführungsform ist die Breite der weiteren Trennwandkanäle 1,5 bis 2,5 Mal so groß wie die Breite der engeren Trennwandkanäle. Bevorzugt liegt die Breite der weiteren Trennwandkanäle im Bereich der 1,5-fachen bis 2,0-fachen Breite der engeren Kanäle, besonders bevorzugt ist sie 1,6 Mal so breit. Das Breitenverhältnis der engeren und weiteren Trennwandkanäle ist abhängig von dem verwendeten Kunststoffmaterial.

In einer bevorzugten Ausführungsform wird das erfindungsgemäße Verfahrens mittels einer zylindrischen Gussform ausgeführt. Die Gussform in Zylinderform dient zur Herstellung eines trommelförmigen, insbesondere zylindrischen Magazins. Bei einer derartigen Gussform fluchtet die Angussöffnung zum Einströmenlassen der Kunststoffmasse mit der Rotationsachse des Magazins. Durch diese zentrale Angussstelle ergibt sich eine gleichmäßige Verteilung der Kunststoffmasse. Die Kunststoffmasse fließt durch die sich radial nach außen erstreckenden Trennwandkanäle zu dem die Mantelfläche des Magazins bildenden Außenwandkanal. Die durch benachbarte Trennwandkanäle strömende Masse trifft dann im Bereich der radial nach außen gerichteten Trennwandkanäle zusammen.

Als vorteilhaft hat sich eine zweiteilige Gussform erwiesen. Besonders bevorzugt ist das erste Teil ein wenigstens einseitig offenes Außenteil, das eine Außenwandung aufweist. Vorzugsweise hat dieses offene Außenteil auch die zentrale Angussstelle zum Einspritzen der Kunststoffmasse in die Gussform. Das zweite Teil ist ein Innenteil, das einen Deckel hat, der die offene Seite des Außenteils abschließt. Selbstverständlich könnte auch das Innenteil über die zentrale Angussstelle verfügen.

Es hat sich als vorteilhaft erwiesen, dass das Innenteil Blöcke umfasst, zwischen denen die Trennwandkanäle ausgebildet werden. Die Blöcke des Innenteils sind so angeordnet, dass sie sich in das Außenteil hinein erstrecken. Jedem Block der Gussform entspricht später eine Kammer des Magazins. In der zylindrischen Gussform zur Herstellung eines (rotationssymmetrischen) Trommelmagazins sind die Blöcke derart angeordnet, dass sich die Trennwandkanäle radial nach außen erstrecken. Bevorzugt haben die Blöcke einen trapezförmigen Querschnitt, wobei sich ihre Weite zur Außenseite der Gussform hin aufweitet (vergrößert).

Selbstverständlich ist es auch möglich, quaderförmige oder anders geformte Magazine mit dem erfindungsgemäßen Verfahren zu erzeugen. Wichtig ist nur, dass die Kunststoffmasse von einer Angussstelle zu einem Außenwandkanal strömt, wobei die in dem Außenwandkanal aushärtende Kunststoffmasse später die Außenwand des Magazins bildet. Bei länglichen Gussformen und somit länglichen Magazinen kann die Angussstelle mit Angussöffnung ebenfalls länglich ausgebildet sein. Auch ist es möglich, mehrere Angussstellen oder mehrere Angussöffnungen vorzusehen. Hierdurch lässt sich ein gleichmäßiges Strömen durch die Trennwandkanäle erzielen, so dass das Zusammenfließen der Kunststoffmassenströme im Bereich der Trennwandkanäle in dem Außenwandkanal gewährleistet ist.

Die in dem erfindungsgemäßen Verfahren verwendete Kunststoffmasse ist nach Aushärtung transparent. In einer bevorzugten Ausgestaltung ist die Kunststoffmasse auch schon im flüssigen bzw. zähfließenden Zustand transparent, also wenn sie erwärmt ist und in die Gussform eingespritzt wird. Der Kunststoff muss so beschaffen sein, dass eine optische Auswertung durch das Kunststoffmaterial hindurch möglich ist.

Bevorzugt wird mit dem erfindungsgemäßen Verfahren ein Magazin hergestellt, dessen Kammern zur Aufnahme von je einem Analyseelement dienen, das eine optisch auswertbare Testzone hat. In der Testzone findet eine optisch messbare Änderung als Reaktion einer Körperflüssigkeit mit einem Reagenz des Analyseelements statt. Die Änderung kann mittels einer optischen, außerhalb des Magazins angeordneten Messeinheit durch die Außenwand der Kammer und somit durch die Außenwand des Magazins gemessen werden. Im Rahmen der Erfindung wurde erkannt, dass die Kunststoffmasse vorzugsweise ein transparenter polymerer Werkstoff sein soll. Besonders bevorzugt wird hierzu ein Cycloolefin-Copolymer eingesetzt. Ein derartiger Werkstoff hat eine hohe Transparenz. Eine optische Messung durch die Außenwand des transparenten Magazins ist somit möglich, ohne dass das Material zu einer Beeinflussung des Messergebnisses führt.

Aufgrund des Verfahrens, bei dem durch benachbarte Trennwandkanäle die Kunststoffmasse mit unterschiedlicher Strömungsgeschwindigkeit fließt und bei dem die Verbindungsnähte in der Außenwand des Magazins mit den Trennwänden zwischen benachbarten Kammern fluchten, ist die Außenwand im Bereich der Kammer frei von Bindenähten. Hierdurch lässt sich eine störungsfreie optische Auswertung eines in den Kammern angeordneten Analyseelements realisieren.

Die Erfindung wird nachfolgend anhand von in den Figuren dargestellten bevorzugten Ausführungsformen näher erläutert. Die darin dargestellten Besonderheiten können einzeln oder in Kombination verwendet werden, um bevorzugte Ausgestaltungen der Erfindung zu schaffen. Die beschriebenen Ausführungsformen stellen keine Einschränkung der Allgemeinheit des in den Ansprüchen definierten Gegenstands dar. Es zeigen:
- Figur 1: eine Schnittdarstellung einer Gussform zur Anwendung des erfindungsgemäßen Herstellungsverfahrens;
- Figur 2: einen Schnitt durch ein erfindungsgemäßes Magazin;
- Figur 3: ein zweiteiliges Magazin, das mit dem erfindungsgemäßen Verfahren hergestellt worden ist; und
- Figur 4: ein Analysegerät mit einem erfindungsgemäßen Magazin für disposible integrierte Stech- und Analyseelemente.

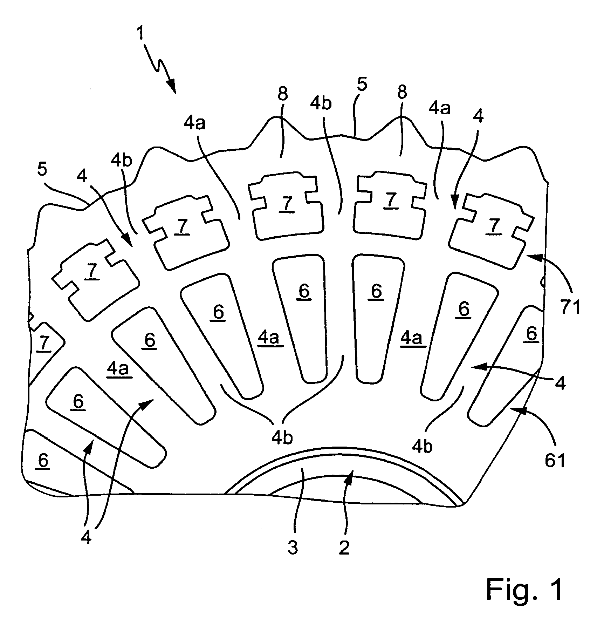

Figur 1 zeigt einen Teilausschnitt eines Schnittbildes durch eine Gussform 1, mittels der ein erfindungsgemäßes Magazin hergestellt werden kann. Die Gussform 1 ist trommelförmig ausgebildet. Eine Angussstelle 2 mit Angussöffnung 3 fluchtet mit der Rotationsachse der Gussform 1. Durch die zentrale Angussöffnung 3 wird eine erwärmte, wenigstens zähfließende Kunststoffmasse in die Gussform 1 eingespritzt. Die Kunststoffmasse breitet sich über Trennwandkanäle 4 aus, die sich radial von der Angussöffnung 3 zu einer Außenwandung 5 der Gussform 1 erstrecken.

Die Trennwandkanäle 4 werden durch Blöcke 6, 7 gebildet, die in zwei ringförmigen Reihen 61, 71 angeordnet sind. Die Blöcke 6 des inneren Rings 61 erstrecken sich radial nach außen und sind im Wesentlichen langgestreckt. Sie bilden in dem Magazin später Kammern, die beispielsweise zur Aufnahme von Trockenmittel vorgesehen sein können oder die leer bleiben und damit das Gesamtgewicht des Magazins reduzieren. Die Blöcke 7 des äußeren Rings 71 weisen eine profilierte Kontur im Querschnitt auf. Sie formen in dem herzustellenden Magazin Kammern zur Aufnahme von Analyseelementen. Zwischen dem äußeren Ring 71 von Blöcken 7 und der Außenwandung 5 ist ein Außenwandkanal 8 gebildet, der die einzelnen Trennwandkanäle 4 miteinander verbindet.

Die Blöcke 6, 7 sind derart angeordnet, dass unterschiedliche breite Trennwandkanäle 4a, 4b gebildet werden, wobei die Kanäle 4a, 4b alternierend angeordnet sind. Die einströmende Kunststoffmasse strömt schneller durch die weiteren Trennwandkanäle 4a als durch die engeren Trennwandkanäle 4b, so dass im Außenwandkanal 8 die Kunststoffmassen derart zusammenfließen, dass die aufgrund des Zusammenfließens gebildete Nahtstelle mit den engeren Trennwandkanälen 4b fluchtet.

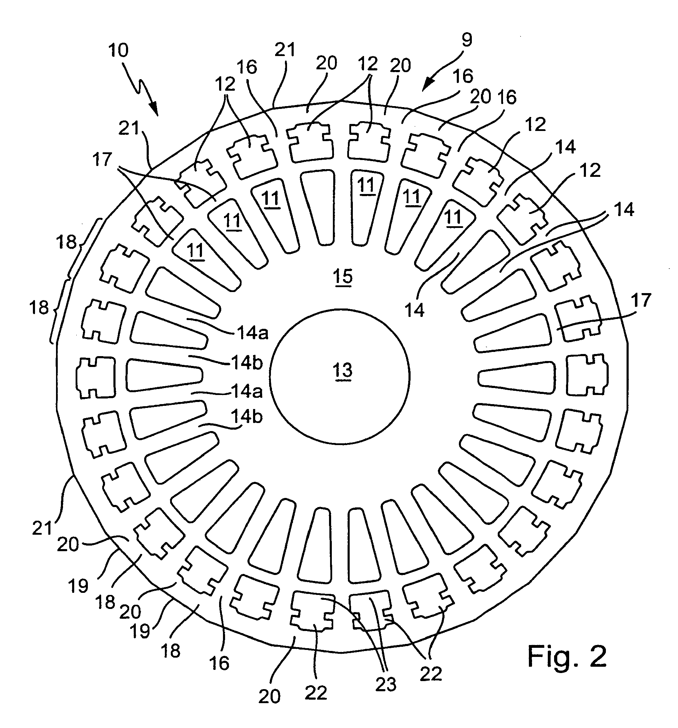

Das Ergebnis des mit der Gussform 1 hergestellten Magazins 10 ist ein in Figur 2 als Schnittdarstellung verdeutlichtes Trommelmagazin 9. Die in der Gussform 1 vorhandenen Blöcke 6, 7 bilden nun die ringförmig angeordneten Kammern 11 in einem inneren Ring und die Kammern 12 in einem äußeren Ring. Durch eine zentrale Rotationsöffnung 13 kann sich eine Welle zur Halterung und Bewegung des Magazins 10 erstrecken.

Zwischen jeweils benachbarten Kammern 11 und jeweils benachbarten Kammern 12 erstrecken sich Trennwände 14 radial nach außen. Die Trennwände 14 verbinden einen inneren Magazinteil 15 (zwischen der Rotationsöffnung 13 und dem aus Kammern 11 gebildeten inneren Ring) mit einer Außenwand 16 des Magazins. Zwischen den inneren Kammern 11 und den (in radialer Richtung fluchtend benachbarten) äußeren Kammern 12 erstrecken sich ringförmig angeordnete Mittelstege 17, die die Trennwände 14 in Umfangsrichtung (also quer zur radialen Richtung) miteinander verbinden.

Die Trennwand 14 bildet somit einen Steg zwischen den jeweils benachbarten Kammern 11 bzw. 12. Jede Trennwand 14 ist eine Seitenwand für zwei Kammern 11, 12 mit zwei gegenüberliegenden Seitenflächen, die jeweils die Seitenfläche einer Kammer 11, 12 bilden. Jede der Kammern 12 hat eine Kammeraußenwand 20, die Teil der Außenwand 16 des Magazins 10 sind. Die Trennwände 14 grenzen an der Kammeraußenwand 20 an.

Die Kammeraußenwände 20 sind bevorzugt gerade. Sie sind Teil eines (ebenen) Außensegments 18, wobei die benachbarten Außensegmente 18 bevorzugt mit einer Bindenaht 21 zusammenstoßen, an der die Kunststoffmassen während des Herstellungsprozesses zusammen sind. Die Bindenähte 21 fluchten hierbei mit den Trennwänden 14 des Magazins 10. Sie sind wenigstens an der äußeren Mantelfläche des Trommelmagazins 9 zu erkennen.

Die Außenwand 16 des Trommelmagazins 9 ist abschnittsweise eben, so dass die Mantelfläche des Trommelmagazins 9 in der hier gezeigten Ausführung keine zylindrische Mantelfläche ist. Die ebenen Außensegmente 18 sind plan ausgeführt, so dass die Magazinaußenflächen 19 der Außensegmente 18 parallel zu den Kammeraußenwänden 20 der Kammern 12 sind. Wie der Figur 2 deutlich zu entnehmen ist, sind je zwei benachbarte Trennwände 14 unterschiedlich breit. Es wechseln sich stets eine weite Trennwand 14a und eine enge Trennwand 14b miteinander ab. Die Bindenähte 21 sind nur im Bereich der engen Trennwände 14b angeordnet. Bevorzugt weist das Magazin 10 eine gradzahlige Anzahl von Kammern 12 auf, in dem gezeigten Beispiel sind sechsundzwanzig Kammern 12 einander benachbart.

Die Kammern 12 sind zur Aufnahme von hier nicht dargestellten Analyseelementen mit einer optisch auswertbaren Testzone geeignet. Die Analyseelemente sind in einer äußeren Teilkammer 22 der Kammer 12 angeordnet. Ihre Testzone ist so ausgerichtet, dass sie der Kammeraußenwand 20 zugewandt ist. Da die Außenwand 16 des Magazins 10, bevorzugt das ganze Magazin 10, transparent ist und die Außenwand 16 im Bereich der Kammeraußenwände 20 frei von Bindenähten 21 ist, ist eine optische Messung durch die Kammeraußenwand 20 möglich, um eine messbare Änderung in der Testzone des Analyseelements zu messen.

Eine zweite Teilkammer 23 der Kammer 12 bildet einen freien Raum, in dem ein Stechelement angeordnet sein oder durch den ein Stechelement hindurch bewegt werden kann.

Die Kammern 11 sind in der hier im Schnitt gezeichneten Ebene des Magazins 10 von den Kammern 12 getrennt. Wenn die Kammern 11 zur Aufnahme von Trocknungsmitteln dienen, sind sie in einer anderen Ebene des Magazins 10 mit der jeweils benachbarten Kammer 12 derart verbunden, dass Feuchtigkeit in den Kammern 12 von dem Trocknungsmittel in den Kammern 11 aufgenommen werden kann. Zwischen den Kammern 11 und 12 besteht folglich eine Luftverbindung.

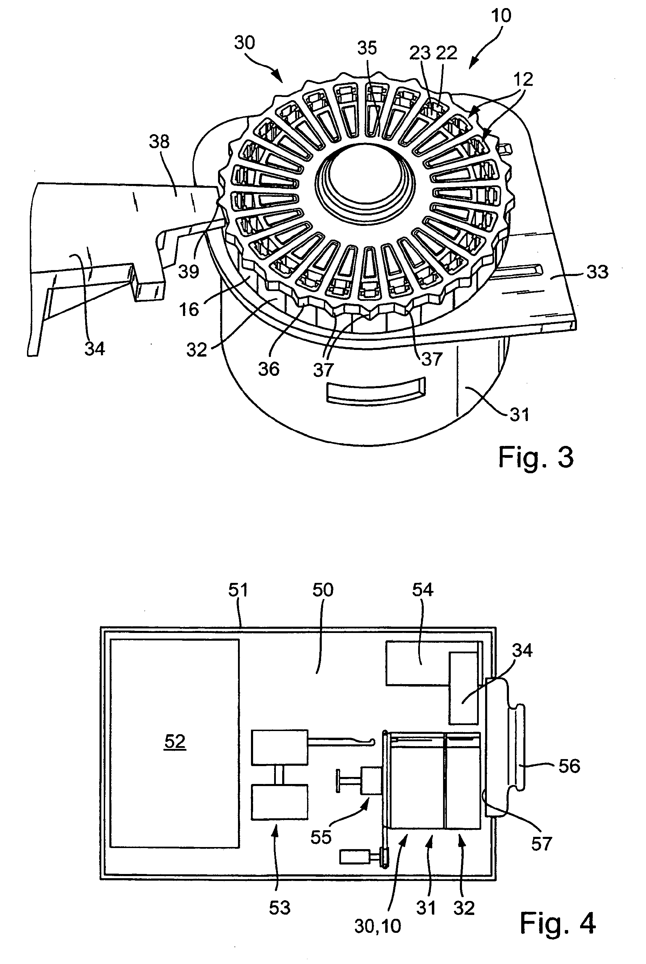

Figur 3 zeigt ein zweiteiliges Trommelmagazin 30 mit einem Stechelement-Teilmagazin 31 und einem Analyseelement-Teilmagazin 32, das das mit dem erfindungsgemäßen Verfahren hergestellte Magazin 10 aus Figur 2 ist. Die beiden Teilmagazine 31, 32 sind in axialer Richtung voneinander beabstandet. Zwischen ihnen ist ein so genanntes Andruckelement 33 angeordnet, das quer zur Axialrichtung des Trommelmagazins 30 verschiebbar ist.

Das Stechelement-Teilmagazin 31 hat einander benachbarte Stechelementkammern, in denen je ein Stechelement enthalten ist. Die Stechelemente sind axial ausgerichtet und können in axialer Richtung derart bewegt werden, dass sie durch das Analyseelement-Teilmagazin 32 bewegt werden und an dessen oberen, freien Stirnseite 35 austreten.

Während der Stechbewegung des Stechelements aus dem Stechelement-Teilmagazin 31 durch das Analyseelement-Teilmagazin 32 wird das Stechelement in der der Rotationsachse nahen Teilkammer 23 der Kammer 12 bewegt. Während der Rückführungsphase der Stechbewegung wird es axial soweit zurückbewegt, bis eine Übergabezone des Stechelements einem in der Teilkammer 22 angeordneten Analyseelement benachbart ist. Eine Bewegung des Andruckelements 33 drückt dann das Stechelement an das Analyseelement an, so dass es zu einem Übertrag der während der Stechbewegung gewonnenen Körperflüssigkeit auf das Analyseelement kommt.

Die übertragene Körperflüssigkeit ruft eine optisch messbare Änderung in dem Analyseelement hervor, die mittels einer photoelektrischen Messeinheit 34 gemessen werden kann. Das Analyseelement-Teilmagazin 32 weist in einem der vorderen Stirnseite 35 benachbarten Bereich einen an der Magazinaußenwand 16 angeordneten Zahnkranz 36 mit einer Mehrzahl von Zähnen 37 auf. Die Zähne 37 dienen als Positionierelemente, um die optische Maßeinheit 34 zuverlässig und reproduzierbar an dem Magazin 10 zu positionieren. Die Maßeinheit 34 weist dazu einen Positionierarm 38 mit einer Positionierkontur 39 auf, die mit den Zähnen 37 korrespondiert.

Für weitere Einzelheiten in Bezug auf das zweiteilige Trommelmagazin 30 wird auf die europäische Patentanmeldung mit der Anmeldenummer EP 08010403 verwiesen, deren Inhalt durch Referenzierung zum Inhalt dieser Anmeldung gemacht wird.

Figur 4 zeigt schließlich das Magazin 10, das in einem Analysegerät 50 mit einem Gehäuse 51, einer Stromversorgung 52, einem Stechantrieb 53 und einer Mess- und Auswerteeinrichtung 54, die die photoelektrische Messeinheit 34 umfasst, angeordnet ist. Eine Halterung 55 dient zur Aufnahme des zweiteiligen Trommelmagazins 30, das so positioniert ist, dass auf der Stirnseite 35 auftretende Stechelemente eine Wunde in einem Körperteil erzeugen können, das an einem Hautkontaktring 56 an einer Gehäuseöffnung 57 anliegt. Nähere Einzelheiten zum Betrieb des Analysegeräts 50 und des Magazins 10, 30 sind der Patentanmeldung mit der Anmeldenummer EP 08010403 zu entnehmen.

### Bezugszeichenliste

- 1: Gussform
- 2: Angussstelle
- 3: Angussöffnung
- 4: Trennwandkanal
- 5: Außenwandung
- 6: Block
- 7: Block
- 8: Außenwandkanal
- 9: Trommelmagazin
- 10: Magazin
- 11,12: Kammer
- 13: Rotationsöffnung
- 14: Trennwand
- 15: innerer Teil
- 16: Außenwand
- 17: Mittelsteg
- 18: Außensegment
- 19: Magazinaußenfläche
- 20: Kammeraußenwand
- 21: Bindenaht
- 22: Teilkammer
- 23: Teilkammer

- 30: zweiteiliges Trommelmagazin
- 31: Stechelement-Teilmagazin
- 32: Analyseelement-Teilmagazin
- 33: Andruckelement
- 34: photoelektrische Messeinheit
- 35: Stirnseite (von 30, 10)
- 36: Zahnkranz
- 37: Zahn
- 38: Positionierarm
- 39: Positionierkontur

- 50: Analysegerät
- 51: Gehäuse
- 52: Stromversorgung
- 53: Stechantrieb
- 54: Mess- und Auswerteeinrichtung
- 55: Halterung
- 56: Hautkontaktring
- 57: Gehäuseöffnung

## Patentansprüche

1. Verfahren zur Spritzgussherstellung eines Magazins (10) mit einer Mehrzahl von Kammern (11, 12), die durch Trennwände (14) voneinander getrennt sind und eine Kammeraußenwand (20) aufweisen, welche von einer Außenwand (16) des Magazins (10) gebildet wird,
mittels einer Gussform (1) mit einem der Außenwand (16) des Magazins (10) entsprechenden Außenwandkanal (8) und den Trennwänden (14) entsprechenden Trennwandkanälen (4) und mit einer Angussöffnung (3), wobei sich mehrere Trennwandkanäle (14) von der Angussöffnung (3) zu dem Außenwandkanal (8) erstrecken,
**gekennzeichnet durch** die folgenden Schritte:
Einspritzen einer Kunststoffmasse in die Angussöffnung (3), die wenigstens nach Aushärtung transparent ist,
Strömenlassen der Kunststoffmasse als Kunststoffmassenstrom von der Angussstelle (3) **durch** die Trennwandkanäle (4) in den Außenwandkanal (8) dergestalt, dass die Kunststoffmassenströme in alternierenden Trennwandkanälen (4) eine unterschiedliche Strömungsgeschwindigkeit haben,
Zusammenfließen der Kunststoffmassenströme in dem Außenwandkanal (8) derart, dass sich an der Außenseite des Außenwandkanals (8) eine Bindenaht (21) ausbildet, die so angeordnet ist, dass sie nicht in der Mitte zwischen zwei benachbarten Trennwandkanälen (4) positioniert ist.

2. Verfahren nach Anspruch 1, **dadurch gekennzeichnet, dass** die Kunststoffmassenströme in dem Außenwandkanal (8) derart zusammenfließen, dass die Bindenaht (21) mit einem Trennwandkanal (4) fluchtet.

3. Verfahren nach Anspruch 1 oder 2, **dadurch gekennzeichnet, dass** benachbarte Trennwandkanäle (4) alternierend weiter und enger sind.

4. Verfahren nach Anspruch 3, **dadurch gekennzeichnet, dass** die Breite der weiteren Trennwandkanäle (4) die 1,1-fache bis 2,5-fache Breite der engeren Trennwandkanäle (4) beträgt, bevorzugt die 1,5-fache bis 2,0-fache Breite, besonders bevorzugt die 1,6-fache Breite.

5. Verfahren nach einem der vorhergehenden Ansprüche, **dadurch gekennzeichnet, dass** die Gussform (1) eine zylindrische Form zur Herstellung eines trommelförmigen Magazins (10) ist und die Angussöffnung (3) mit der Rotationsachse des Magazins (10) fluchtet.

6. Verfahren nach einem der vorhergehenden Ansprüche, **dadurch gekennzeichnet, dass** die Gussform (1) zweiteilig ist, wobei bevorzugt
- ein wenigstens einseitig offenes Außenteil eine Außenwandung (5) und die zentrale Angussstelle (2) aufweist, und
- ein Innenteil einen Deckel hat, der die offene Seite des Außenteils abschließt.

7. Verfahren nach Anspruch 6, **dadurch gekennzeichnet, dass** das Innenteil Blöcke (6, 7) umfasst, zwischen denen die Trennwandkanäle (4) gebildet werden, wobei sich die Blöcke (6, 7) des Innenteils in das Außenteil erstrecken.

8. Verfahren nach einem der vorhergehenden Ansprüche, **dadurch gekennzeichnet, dass** die Kammern (12) zur Aufnahme von je einem Analyseelement dienen, das eine optisch auswertbare Testzone hat, wobei eine in der Testzone stattfindende Änderung mittels einer photoelektrischen Messeinheit (34) durch die Kammeraußenwand (20) der Kammer (12) hindurch optisch gemessen werden kann.

9. Verfahren nach einem der vorhergehenden Ansprüche, **dadurch gekennzeichnet, dass** die Kunststoffmasse ein transparenter polymerer Werkstoff ist, bevorzugt ein Cycloolefin-Copolymer.

10. Magazin für ein Analysegerät mit Kammern,
wobei die Kammern (12) benachbart zueinander angeordnet sind und eine Kammeraußenwand (20) aufweisen, die Teil einer Außenwand (16) des Magazins (10) ist,
die Kammern (12) zwei Trennwände (14) aufweisen,
das Magazin (10) mittels Spritzgussverfahren hergestellt ist,
**dadurch gekennzeichnet, dass**
das Magazin (10) mittels eines Verfahrens nach Anspruch 1 herstellbar ist,
die Außenwand (16) des Magazins (10) im Bereich der Kammeraußenwände (20) transparent ist,
das Magazin (10) an der Außenwand (16) Bindenähte (21) aufweist, wobei die Bindenähte (21) außerhalb der Mitte der sich zwischen zwei benachbarten Trennwänden (14) erstreckenden Kammeraußenwand (20).

11. Magazin nach Anspruch 10, **dadurch gekennzeichnet, dass** die Bindenähte (21) mit den Trennwänden (14) der Kammern (12) fluchten.

12. Magazin nach Anspruch 10 oder 11, **dadurch gekennzeichnet, dass** jede Kammer (12) zwei unterschiedlich dicke Trennwände (14) hat.

13. Magazin nach einem der Ansprüche 10 bis 12, **dadurch gekennzeichnet, dass** das Magazin (10) ein Trommelmagazin (9) ist.

14. Magazin nach einem der Ansprüche 11 bis 13, **dadurch gekennzeichnet, dass** die Kammeraußenwände (20) transparent und frei von Bindenähten (21) sind.

15. Magazin nach einem der Ansprüche 10 bis 14, **dadurch gekennzeichnet, dass** die Außenwand (16) des Magazins (10) transparent ist.

16. Magazin nach einem der Ansprüche 10 bis 15, **dadurch gekennzeichnet, dass** die Kammern (12) zur Aufnahme von Analyseelementen mit einer optisch auswertbaren Testzone geeignet sind, wobei an der Testzone eine optisch messbare Änderung als Reaktion einer Körperflüssigkeit mit einem Reagenz des Analyseelements stattfindet und die Änderung mittels einer photoelektrischen Messeinheit (34) durch die Kammeraußenwand (20) der Kammer (12) hindurch optisch gemessen werden kann.

## Claims

1. A method for the injection-molding production of a magazine (10) having a plurality of chambers (11, 12), which are separated from one another by partition walls (14) and have a chamber outer wall (20), which is formed by an outer wall (16) of the magazine (10),
using a casting mold (1) having an outer wall channel (8), which corresponds to the outer wall (16) of the magazine (10), and partition wall channels (4), which correspond to the partition walls (14), and having a gate opening (3), a plurality of partition wall channels (14) extending from the gate opening (3) to the outer wall channel (8).
**characterized by** the following steps:
injecting a plastic compound, which is transparent at least after curing, into the gate opening (3),
causing the plastic compound to flow as a plastic compound stream from the gate point (3) through the partition wall channels (4) into the outer wall channel (8) in such a manner that the plastic compound streams have a differing flow speed in alternating partition wall channels (4),
flowing together of the plastic compound streams in the outer wall channel (8) in such a manner that a joint line (21) forms on the outer side of the outer wall channel (8), which is positioned so that it is not positioned in the center between two adjacent partition wall channels (4).

2. Method according to claim 1, **characterized in that** the plastic compound streams flow together in the outer wall channel (8) in such a manner that the joint line (21) is aligned with a partition wall channel (4).

3. Method according to claim 1 or 2, **characterized in that** adjacent partition wall channels (4) are alternately wider and narrower.

4. Method according to claim 3, **characterized in that** the width of the wider partition wall channels (4) is 1.1 times to 2.5 times the width of the narrower partition wall channels (4), preferably 1.5 times to 2.0 times the width, particularly preferably 1.6 times the width.

5. Method according to any one of the preceding claims, **characterized in that** the casting mold (1) is a cylindrical mold for producing a drum-shaped magazine (10) and the gate opening (3) is aligned with the rotational axis of the magazine (10).

6. Method according to any one of the preceding claims, **characterized in that** the casting mold (1) is in two parts, preferably
- an outer part, which is open at least on one side, having an outer wall (5) and the central gate point (2), and
- an inner part having a lid, which terminates the open side of the outer part.

7. Method according to claim 6, **characterized in that** the inner part comprises blocks (6, 7), between which the partition wall channels (4) are formed, the blocks (6, 7) of the inner part extending into the outer part.

8. The method according to any one of the preceding claims, **characterized in that** the chambers (12) are each used to receive one analysis element, which has an optically analyzable test zone, a change occurring in the test zone being able to be optically measured through the chamber outer wall (20) of the chamber (12) by means of a photoelectric measuring unit (34).

9. Method according to any one of the preceding claims, **characterized in that** the plastic compound is a transparent polymer material, preferably a cycloolefin copolymer.

10. Magazine for an analysis device having chambers,
the chambers (12) being positioned adjacent to one another and having a chamber outer wall (20), which is part of an outer wall (16) of the magazine (10),
the chambers (12) having two partition walls (14),
the magazine (10) being produced using injection-molding methods, **characterized in that**
the magazine (10) being producable using a method according to claim 1,
the outer wall (16) of the magazine (10) being transparent in the area of the chamber outer walls (20),
the magazine (10) having joint lines (21) on the outer wall (16), the joint lines (21) being positioned outside the middle of the chamber outer wall (20), which extends between two adjacent partition walls (14).

11. Magazine according to claim 10, **characterized in that** the joint lines (21) are aligned with the partition walls (14) of the chambers (12).

12. Magazine according to claim 10 or 11, **characterized in that** each chamber (12) has two partition walls (14) of different thicknesses.

13. Magazine according to any one of claims 10 to 12, **characterized in that** the magazine (10) is a drum magazine (9).

14. Magazine according to any one of claims 11 to 13, **characterized in that** the chamber outer walls (20) are transparent and free of joint lines (21).

15. Magazine according to any one of claims 10 to 14, **characterized in that** the outer wall (16) of the magazine (10) is transparent.

16. Magazine according to any one of claims 10 to 15, **characterized in that** the chambers (12) are suitable for receiving analysis elements having an optically analyzable test zone, an optically measurable change occurring on the test zone as a reaction of a body fluid with a reagent of the analysis element and the change being able to be measured through the chamber outer wall (20) of the chamber (12) by means of a photoelectric measuring unit (34).

## Revendications

1. Procédé pour fabriquer, par moulage par injection, un magasin (10) comprenant une pluralité de chambres (11, 12) qui sont séparées les unes des autres par des parois de séparation (14) et qui présentent une paroi extérieure (20) formée par une paroi extérieure (16) du magasin (10),
au moyen d'un moule (1) comprenant un canal de paroi extérieure (8) correspondant à la paroi extérieure (16) du magasin (10) et des canaux de parois de séparation (4) correspondant aux parois de séparation (14) ainsi qu'un orifice d'injection (3), plusieurs canaux de parois de séparation (14) s'étendant de l'orifice d'injection (3) vers le canal de paroi extérieure (8),
**caractérisé par** les étapes suivantes ;
injecter une masse de matière plastique dans l'orifice d'injection (3), laquelle masse est transparente au moins une fois durcie,
faire s'écouler la masse de matière plastique sous forme de flux massique de matière plastique du point d'injection (3) à travers les canaux de parois de séparation (4) jusque dans le canal de paroi extérieure (8), de sorte que les flux massiques de matière plastique aient une vitesse d'écoulement différente dans des canaux de parois de séparation (4) alternés,
faire confluer les flux massiques de matière plastique dans le canal de paroi extérieure (8), de sorte qu'un cordon de jonction (21) se forme sur le côté extérieur du canal de paroi extérieure (8), dont la disposition est telle qu'il ne se trouve pas positionné au milieu entre deux canaux de parois de séparation (4) adjacents.

2. Procédé selon la revendication 1, **caractérisé en ce que** les flux massiques de matière plastique confluent dans le canal de paroi extérieure (8), de sorte que le cordon de jonction (21) se trouve aligné avec un canal de paroi de séparation (4).

3. Procédé selon la revendication 1 ou 2, **caractérisé en ce que** les canaux de parois de séparation (4) adjacents sont, de manière alternée, plus larges et plus étroits.

4. Procédé selon la revendication 3, **caractérisé en ce que** la largeur des canaux de parois de séparation (4) plus larges représente 1,1 à 2,5 fois la largeur des canaux de parois de séparation (4) plus étroits, de préférence 1,5 à 2,0 fois, de manière particulièrement préférée 1,6 fois cette largeur.

5. Procédé selon l'une des revendications précédentes, **caractérisé en ce que** le moule (1) est un moule cylindrique pour la fabrication d'un magasin (10) en forme de tambour et **en ce que** l'orifice d'injection (3) est aligné avec l'axe de rotation du magasin (10).

6. Procédé selon l'une des revendications précédentes, **caractérisé en ce que** le moule (1) est en deux parties dont, de manière préférentielle,
- une partie extérieure, ouverte au moins sur un côté, présente une paroi extérieure (5) et le point d'injection (2) central, et
- une partie intérieure possède un couvercle qui ferme le côté ouvert de la partie extérieure.

7. Procédé selon la revendication 6, **caractérisé en ce que** la partie intérieure comprend des blocs (6, 7) entre lesquels les canaux de parois de séparation (4) seront formés, lesdits blocs (6, 7) de la partie intérieure s'étendant jusque dans la partie extérieure.

8. Procédé selon l'une des revendications précédentes, **caractérisé en ce que** les chambres (12) servent à recevoir respectivement un élément d'analyse doté d'une zone de test susceptible d'être exploitée optiquement, un changement intervenant dans ladite zone de test pouvant être mesuré optiquement à travers la paroi extérieure (20) de la chambre (12) au moyen d'un ensemble de mesure photoélectrique (34).

9. Procédé selon l'une des revendications précédentes, **caractérisé en ce que** la masse de matière plastique est un matériau polymère transparent, de préférence un copolymère de cyclooléfine.

10. Magasin pour un appareil d'analyse comprenant des chambres,
les chambres (12) étant disposées de manière adjacente les unes par rapport aux autres et ayant une paroi extérieure (20) qui fait partie d'une paroi extérieure (16) du magasin (10),
les chambres (12) ayant deux parois de séparation (14),
le magasin (10) étant fabriqué au moyen d'un procédé de moulage par injection,
**caractérisé en ce que**
le magasin (10) peut être fabriqué au moyen d'un procédé selon la revendication 1,
la paroi extérieure (16) du magasin (10) est transparente au niveau des parois extérieures de chambres (20),
le magasin (10) présente sur la paroi extérieure (16) des cordons de jonction (21), lesdits cordons de jonction (21) étant excentrés par rapport à la paroi extérieure de chambre (20) s'étendant entre deux parois de séparation (14) adjacentes.

11. Magasin selon la revendication 10, **caractérisé en ce que** les cordons de jonction (21) sont alignés avec les parois de séparation (14) des chambres (12).

12. Magasin selon la revendication 10 ou 11, **caractérisé en ce que** chaque chambre (12) a deux parois de séparation (14) d'épaisseur différente.

13. Magasin selon l'une des revendications 10 à 12, **caractérisé en ce que** le magasin (10) est un magasin à tambour (9).

14. Magasin selon l'une des revendications 11 à 13, **caractérisé en ce que** les parois extérieures de chambres (20) sont transparentes et exemptes de cordons de jonction (21).

15. Magasin selon l'une des revendications 10 à 14, **caractérisé en ce que** la paroi extérieure (16) du magasin (10) est transparente.

16. Magasin selon l'une des revendications 10 à 15, **caractérisé en ce que** les chambres (12) sont adaptées à recevoir des éléments d'analyse dotés d'une zone de test susceptible d'être exploitée optiquement, un changement mesurable optiquement ayant lieu dans ladite zone de test en tant que réaction d'un liquide corporel avec un réactif de l'élément d'analyse et le changement pouvant être mesuré optiquement à travers la paroi extérieure (20) de la chambre (12) au moyen d'un ensemble de mesure photoélectrique (34).
